# EUROPEAN PATENT SPECIFICATION

(11) **EP 2 596 813 B1**
(45) Date of publication and mention of the grant of the patent: **05.09.2018**
(21) Application number: 11809600.7
(22) Date of filing: 14.07.2011
(51) Int. Cl.: A61L 24/00, A61K 9/70, A61K 38/43, A61K 38/48, A61K 47/34, A61L 26/00, A61K 38/36, A61F 13/00, A61L 15/26, A61L 15/32

(54) **SHEET PREPARATION FOR TISSUE ADHESION**
FOLIENPRÄPARAT FÜR GEWEBEADHÄSION
PRÉPARATION DE FEUILLE POUR L'ADHÉSION DE TISSU

(30) Priority: 20.07.2010 JP 2010162979
(43) Date of publication of application: 29.05.2013
(73) Proprietor: The Chemo-Sero-Therapeutic Research Institute, Kumamoto-shi Kumamoto 860-8568 (JP)
(72) Inventor: SAGA, Hideki, Kumamoto-shi Kumamoto 860-8568 (JP); HAMURO, Tsutomu, Kumamoto-shi Kumamoto 860-8568 (JP)
(74) Representative: Vossius & Partner Patentanwälte Rechtsanwälte mbB
(86) International application number: PCT/JP2011/066099
(87) International publication number: WO 2012/011429

(56) References cited:
- EP-A1- 1 588 722
- EP-A1- 1 759 718
- WO-A1-97/37694
- WO-A1-2005/113030
- JP-A- 2004 520 124
- JP-A- 2009 183 649
- JP-A- 2009 183 649
- JP-A- 2009 545 423
- TAKAO OASA ET AL.: 'Tacho Comb Case Report Keidomyaku Shujutsu ni Okeru Tacho Comb no Yuyosei' IYAKU NO MON vol. 42, no. 2, 2002, pages 108 - 109, XP008169426
- YOTARO IZUMI ET AL.: 'Idenshi Kumikae Thrombin Tofu Sheet Seizai no Haidomyaku Shukketsu ni Taisuru Shiketsu Koka' DAI 60 KAI THE JAPANESE ASSOCIATION FOR THORACIC SURGERY TEIKI GAKUJUTSU SHUKAI 2007, page 150 (065), XP008169267
- GUENTHER WITZKE: 'Tacho Comb no Rinsho Oyo: Geka Shujutsu ni Okeru Shiyoho ni Kansuru Tenbo' THE JOURNAL OF MEDICINE vol. 54, no. 4, 2005, pages 469 - 483, XP008169252

## Description

### TECHNICAL FIELD

The present invention relates to a sheet preparation consisting of a sheet-shaped support which comprises fibrinogen and thrombin as an active ingredient and a process for preparing the same. More specifically, the present invention relates to a sheet preparation used for tissue adhesion characterized by that fibrinogen is held on one surface of a sheet-shaped support and thrombin is held on the other surface of the sheet-shaped support, and a process for preparing the same.

### BACKGROUND ART

Fibrinogen is a very important coagulation factor which acts in the final stage of the blood coagulation cascade. Fibrinogen, e.g. upon activation of the coagulation system after an injury, is converted by thrombin from its soluble form into insoluble fibrin which plays an important role in hemostasis and wound healing.

Fibrinogen has importance in hemostasis and wound healing. For instance, fibrinogen has been used clinically as an intravenous dosage form in a replacement therapy against congenital and acquired fibrinogen deficiencies etc. to hamper a serious bleeding by increasing the fibrinogen level in blood. Additionally, in recent years, a fibrin adhesive consisting of fibrinogen and thrombin is used in a surgery as an adhesive for substitute of suture of soft organs such as the liver and the spleen or as an auxiliary agent for the suture. Fibrinogen has also widely been applied in other clinical set-up.

Such a fibrin adhesive, capable of adhering to a wound or a tissue surface, may enhance a tension strength of an adhesion site or a joined wound, may fully be absorbed within the living body and may promote the healing of wound.

A fibrin adhesive is not stable in a form of a solution and thus is used in clinical practice in a dosage form of a frozen solution or a lyophilized powder. Therefore, a commercially available preparation has to be thawed or rehydrated before application, in either of which a lot of time is wasted. Specifically, a fibrin adhesive that is prepared from a lyophilized product consists of a lyophilized powder of fibrinogen, a fibrinogen solution, a lyophilized powder of thrombin and a thrombin solution. When it takes time for dissolving the lyophilized powder and for preparing for applying and mixing the fibrinogen solution and the thrombin solution, there are concern that it might adversely affect a patient, especially in case of a surgical operation at emergency.

Additionally, to obtain a sufficient adhesive action as a fibrin adhesive, it is necessary to dissolve fibrinogen in a higher concentration. To be coagulated fully, the higher concentration of fibrinogen is the more favorable. However, there is a problem that such a high-concentration fibrinogen solution is not suitable for use in a surgical operation at emergency since it would take a long time to make the solution from a lyophilized fibrinogen preparation.

A sheet preparation consisting of a bioabsorbable sheet holding thrombin, and a fibrinogen solution is reported (Patent reference 1, Patent reference 2). The sheet preparation is used in clinical set-up of a surgical operation such that a sheet holding thrombin is immersed in a fibrinogen solution and then applied to the operation site. The sheet preparation may exert excellent hemostatic and adhesive effects. However, although thrombin is held on a sheet in advance, the sheet preparation may still not be used for emergent use since it would take time for preparing a fibrinogen solution.

Thus, a pharmaceutical preparation being looked for in clinical set-up is (1) a pharmaceutical preparation having high adhesive and hemostatic effects; (2) a dosage form that does not require time for preparation, i.e. an integrated sheet preparation in which fibrinogen and thrombin are held together. An integrated sheet preparation currently approved for clinical use includes a sheet preparation (TachoComb (registered trademark): CSL Behring) in which fibrinogen and thrombin are mixed and held on a support made of equine collagen. However, efficacy of said sheet preparation is limited and does not satisfy surgeons. Furthermore, a process for preparing said sheet preparation is extremely complicated (Patent reference 3). Thus, in clinical set-up, a pharmaceutical preparation is desired that can cope with surgical operation at emergency and has potent tissue-adhesive, sealing and hemostatic effects. Besides, inventing a more convenient process for preparing a sheet preparation would allow for provision of an inexpensive pharmaceutical preparation with potent tissue-adhesive, sealing and hemostatic effects in clinical set-up.

A sheet-shaped fibrin adhesive consisting of two-layered bioabsorbable supports, one layer holding fibrinogen as an effective ingredient and the other layer holding thrombin as an effective ingredient, is known (Patent reference 4). However, since said invention is consisted of two-layered supports, its handling for applying to the affected parts is troublesome and, in case of a microscopic operation, its application to the affected parts would be difficult. Besides, in case of emergent operation or irregular hemorrhage, it is also difficult to apply.

A carrier having at least one of physical properties of ventricles, i.e. an elastic module in a range of 5-100 N/cm, a density of 1-10 mg/cm³, a diameter of more than 0.75 mm and less than 4 mm, and/or a mean diameter of less than 3 mm, and a solid composition comprising said carrier that holds solid fibrinogen and solid thrombin are known (Patent reference 5). However, since said invention is not a sheet preparation, it is difficult to use the composition with pressure. In addition, said composition has a low tensile strength and a poor tissue sealing capacity. Besides, its handling is troublesome since an applicator is necessary for applying to the affected parts. Furthermore, as a consequence of holding of solid fibrinogen and solid thrombin on a carrier, the two ingredients may instantaneously react with each other to form fibrin so that insufficient adhesion to the tissue to be applied would result. For exerting a high adhesive effect, it is critical that fibrinogen, while penetrating into a tissue adhesion site, is reacted with thrombin to gelation.
Patent reference 1: WO 2004/064878
Patent reference 2: WO 2005/113030
Patent reference 3: Japanese Patent Publication No. 2004-520124
Patent reference 4: Japanese Patent Publication No. 2010-69031
Patent reference 5: Japanese Patent Publication No. 2007-190399

JP 2009 183649 A relates to a sheet like tissue adhesive comprising a fibrinogen layer, a thrombin layer and an intermediate layer located between the fibrinogen layer and the thrombin layer.

### DISCLOSURE OF THE INVENTION

### (Technical Problem to be Solved by the Invention)

Under these circumstances, an object of the present invention is to provide (1) a pharmaceutical preparation having high adhesive and hemostatic effects; (2) a dosage form that does not require time for preparation; (3) a pharmaceutical preparation that can easily be prepared and is inexpensive, i.e. an integrated sheet preparation in which fibrinogen and thrombin are held together, and a process for preparing the same. Specifically, an object of the present invention is to provide a sheet preparation that may exert more potent tissue-adhesive, sealing and hemostatic effects than the currently approved pharmaceutical preparations and is inexpensive, and a process for preparing the same.

### (Means for Solving the Problems)

Thus, viewing the above problems, the present inventors have earnestly studied and as a result found that an integrated sheet preparation in which fibrinogen and thrombin are held together possesses the above properties, which integrated sheet preparation is prepared by soaking one surface of a sheet-shaped support with a fibrinogen solution and lyophilizing the sheet-shaped support to thereby let a lyophilized powder of fibrinogen be held on the sheet-shaped support, and then applying a powder dispersion of a thrombin powder to the other surface of the sheet-shaped support and drying the sheet-shaped support to thereby let a dry powder of thrombin be held on the sheet-shaped support. Said sheet preparation may hold fibrinogen at a high concentration per area of the surface that holds fibrinogen through holding a lyophilized dry powder of fibrinogen on the surface. Thus, said sheet preparation may exert more potent tissue-adhesive, sealing and hemostatic effects than the currently available pharmaceutical preparations. The present invention is explained in more detail hereinbelow.

The present invention includes the following features.
[1] A sheet preparation characterized by that fibrinogen is held on one surface of a sheet-shaped support and thrombin is held on the other surface of the sheet-shaped support where fibrinogen is not held and that fibrinogen and thrombin are held separately from each other, wherein fibrinogen is a lyophilized powder of fibrinogen which is obtainable by applying or soaking a fibrinogen solution to one surface of the sheet-shaped support and then lyophilizing the sheet-shaped support, wherein said fibrinogen solution is one that contains 3% to 6% (w/v) fibrinogen, and wherein the sheet-shaped support is one made from polyglycolic acid and having a thickness of at least 0.3 mm.
[2] The sheet preparation according to the above [1] wherein said sheet-shaped support is one made from a non-woven fabric prepared by needle-punching a knit or a textile of polyglycolic acid.
[3] The sheet preparation according to any of the above [1] or [2] wherein said fibrinogen is held on the surface of the sheet-shaped support at 1.5 mg/cm² or more per area of the surface that holds fibrinogen.
[4] The sheet preparation according to any of the above [1] to [3] wherein thrombin is a thrombin powder.
[5] The sheet preparation according to the above [4] wherein said thrombin powder is one prepared by applying a powder dispersion in which a thrombin powder is dispersed in a solvent to the other surface of the sheet-shaped support where fibrinogen is not held and drying the sheet-shaped support.
[6] The sheet preparation according to the above [5] wherein said solvent is alcohols.
[7] The sheet preparation according to the above [6] wherein said alcohols are selected from the group consisting of ethanol, methanol, isopropyl alcohol, 1-butanol, 2-butanol, isobutyl alcohol, and isopentyl alcohol.
[8] The sheet preparation according to the above [7] wherein said alcohols are ethanol.
[9] The sheet preparation according to any of the above [1] to [8] wherein said thrombin is held on the surface of the sheet-shaped support at 0.28 U/cm² or more per area of the surface that holds thrombin.
[10] The sheet preparation according to any of the above [1] to [9] wherein said sheet preparation comprises at least one selected from the group consisting of Factor XIII, aprotinin, albumin, calcium chloride, a nonionic surfactant, and various amino acids.
[11] A process for preparing the sheet preparation as set forth in any of the above [1] to [10], said process comprising holding fibrinogen on one surface of a sheet-shaped support and holding thrombin on the other surface of the sheet-shaped support where fibrinogen is not held.
[12] A process for preparing a sheet preparation according to the above [11] which comprises the steps:
   (A) applying or soaking one surface of a sheet-shaped support with a fibrinogen solution and lyophilizing the sheet-shaped support to thereby let a lyophilized powder of fibrinogen be held on the sheet-shaped support, and
   (B) applying a powder dispersion of a thrombin powder to the other surface of the sheet-shaped support and drying the sheet-shaped support to thereby let a thrombin powder be held on the sheet-shaped support;
   wherein fibrinogen and thrombin are separately held on the distinct surfaces of the sheet-shaped support.
[13] The process for preparing a sheet preparation according to the above [12] wherein said fibrinogen solution is applied in such an amount that said fibrinogen solution does not reach the other surface of the sheet-shaped support where a thrombin powder is held.
[14] The sheet preparation as set forth in any of the above [1] to [10] for use in hemostasis.

### EFFECTS OF THE INVENTION

In accordance with the present invention, a sheet preparation with a lyophilized fibrinogen which holds a thrombin powder and a process for preparing the same are provided. The sheet preparation according to the present invention may exert excellent effects as follows:
(1) It may exert more potent tissue-adhesive, sealing and hemostatic effects than the currently available pharmaceutical preparations;
(2) It may form a γ-dimer, which the currently available pharmaceutical preparations cannot form, to thereby enhance physical strength and stability of fibrin clot;
(3) It is a very convenient pharmaceutical preparation that does not require time for preparation and may be used in case of emergency;
(4) It is excellent in rehydration;
(5) It is bioabsorbable;
(6) It is excellent in flexibility and elasticity;
(7) It is excellent in holding the effective ingredients; and
(8) It can easily be prepared and is inexpensive.

### BRIEF DESCRIPTION OF DRAWINGS

Fig. 1 shows results of a test for evaluating an adhesive effect of the sheet preparation of the present invention.
Fig. 2 shows results of a test for evaluating an adhesive effect of the sheet preparation of the present invention.
Fig. 3 shows results of a test for evaluating an adhesive effect of the sheet preparation of the present invention.
Fig. 4 shows results of SDS-PAGE of the sheet preparation of the present invention.
Fig. 5 shows results of a test for evaluating a hemostatic effect of the sheet preparation of the present invention.

### BEST MODE FOR CARRYING OUT THE INVENTION

The sheet preparation of the present invention is one characterized by that fibrinogen is held on one surface of a sheet-shaped support and thrombin is held on the other surface of the sheet-shaped support where fibrinogen is not held and that fibrinogen and thrombin are held separately from each other.

Fibrinogen is a lyophilized powder. In addition to fibrinogen, fibrinogen may further include Factor XIII, aprotinin, albumin, sodium chloride, sodium citrate, a nonionic surfactant such as Polysorbate 80, various amino acids, glycerol, and the like.

In a process for holding fibrinogen, a fibrinogen solution may be applied or soaked to one of the surfaces of a sheet-shaped support which is then lyophilized so as to hold a lyophilized powder of fibrinogen or alternatively a lyophilized powder of fibrinogen may directly be applied to one of the surfaces of a sheet-shaped support. Preferably, a fibrinogen solution may be soaked to a sheet-shaped support which is then lyophilized. Under these circumstances, fibrinogen should not be applied, infiltrated or presented to the other surface of a sheet-shaped support.

Fibrinogen may preferably be held on the surface of a sheet-shaped support at 1.5 mg/cm² or more per area of the surface that holds fibrinogen, more preferably at 1.5 mg/cm² to 30 mg/cm², even more preferably at 1.5 mg/cm² to 15 mg/cm², even more preferably at 1.5 mg/cm² to 12 mg/cm², even more preferably at 3 mg/cm² to 12 mg/cm², and even more preferably at 6 mg/cm² to 12 mg/cm². An amount of fibrinogen to be held on a sheet-shaped support may suitably be varied depending on a thickness of a sheet-shaped support or a concentration of a fibrinogen solution.

The fibrinogen solution contains fibrinogen at 3% to 6% (w/v). A concentration of fibrinogen may suitably be varied depending on a thickness of a sheet-shaped support or an amount of fibrinogen to be held on a sheet-shaped support.

Thrombin may be a dried powder, a lyophilized powder, or a gel containing thrombin, preferably a dried powder. In addition to thrombin, thrombin may further include albumin, sodium chloride, sodium citrate, a nonionic surfactant such as Polysorbate 80, various amino acids, glycerol, and the like.

In a process for holding thrombin, a powder dispersion of a thrombin powder may be applied to a sheet-shaped support which is then dried or alternatively a thrombin powder may directly be applied to a sheet-shaped support. Preferably, a powder dispersion of a thrombin powder may be applied to a sheet-shaped support which is then dried to hold thrombin.

A thrombin powder may be applied to a sheet-shaped support at 0.28 U/cm² or more per area of the surface that holds thrombin, more preferably at 0.28 to 60 U/cm², even more preferably at 0.28 to 30 U/cm², even more preferably at 0.28 to 15 U/cm², even more preferably at 0.28 to 13.8 U/cm², even more preferably at 1.38 to 13.8 U/cm², and even more preferably at 4.6 to 13.8 U/cm². An amount of thrombin to be held on a sheet-shaped support may suitably be varied depending on an amount of fibrinogen to be held on a sheet-shaped support.

For a powder dispersion of a thrombin powder, an organic solvent may preferably be used as a main solvent. Preferably, an alcohol solvent such as ethanol, methanol, isopropyl alcohol, 1-butanol, 2-butanol, isobutyl alcohol, or isopentyl alcohol, or a solvent with a low boiling point that does not affect the human body may be used as a main solvent. In view of safety to the human body, ethanol may preferably be used as a main solvent.

A powder dispersion of a thrombin powder may preferably contain 5.6 U/mL to 1380 U/mL of thrombin, and 0.064 mg/mL to 64 mg/mL of calcium chloride. More preferably, it contains 27.6 U/mL to 276 U/mL of thrombin, and 0.64 mg/mL to 6.4 mg/mL of calcium chloride. A concentration of a thrombin powder may suitably be varied depending on a thickness of a sheet-shaped support or an amount of thrombin to be held on a sheet-shaped support.

For an order of fibrinogen and thrombin to be held on a sheet-shaped support, fibrinogen may firstly be held on a sheet-shaped support and thereafter thrombin may be held or alternatively thrombin may firstly be held on a sheet-shaped support and thereafter fibrinogen may be held, provided that fibrinogen and thrombin do not contact to, and react with, each other before application. In view of convenience and easiness in production, however, fibrinogen may preferably firstly be held on a sheet-shaped support and thereafter thrombin may be held. Specifically, one of the surfaces of a sheet-shaped support may be immersed in a fibrinogen solution to soak the fibrinogen solution and then the sheet-shaped support may be lyophilized to prepare a sheet holding fibrinogen. While preparation, it is necessary to adjust an amount of a fibrinogen solution to one that does not reach the other surface of a sheet-shaped support where a thrombin powder is held, taking into consideration an area and a thickness of a sheet-shaped support to be used. Thereafter, a powder dispersion of a thrombin powder dispersed in ethanol may be applied to the surface of a sheet-shaped support opposite to the surface where fibrinogen is held and the sheet-shaped support may be dried. The thickness of a sheet-shaped support is at least 0.3 mm, even more preferably at least 0.4 mm, even more preferably at least 0.8 mm, and even more preferably at least 1 mm. An upper limit of a thickness of a sheet-shaped support may suitably be varied depending on the tissue to which the sheet preparation is applied and an amount of fibrinogen to be applied. The bioabsorbable material used for the sheet-shaped support is polyglycolic acid.

A structure of a sheet-shaped support may be in the form of nonwoven fabric, sponge, textile, knit, braid or a composite thereof, among which nonwoven fabric is preferable.

Nonwoven fabric may preferably be one manufactured by, for instance, meltblown, needlepunching, spunbonding, flash spinning, and the like. Especially, preferable is nonwoven fabric manufactured by needle-punching piled woven or knitted fabric using bioabsorbable material (e.g. polyglycolic acid) into nonwoven fabric (Japanese Patent Publication No. 18579/1993).

A method for preparing fibrinogen, thrombin, albumin, aprotinin and Factor XIII as used herein is not particularly limited and includes e.g. separation from human blood or by genetic recombination technique.

The present invention is explained in more detail by means of the following Examples but is not limited thereto.

### EXAMPLE

### Example 1: Process for preparing sheet preparation

For a sheet-shaped support, a bioabsorbable sheet was used. Specifically, for a bioabsorbable sheet, Neoveil 015G (registered trademark) (Gunze; composition: polyglycolic acid; thickness 0.15 mm), Neoveil 03G (registered trademark) (Gunze; composition: polyglycolic acid; thickness 0.3 mm), and Neoveil 05G (registered trademark) (Gunze; composition: polyglycolic acid; thickness 0.5 mm) were used.

A fibrinogen solution I was prepared to contain about 12.0% (w/v) fibrinogen, about 90 IU/mL Factor XIII, 1.5% (w/v) human serum albumin, 2.6% (w/v) sodium chloride, 1.8% (w/v) trisodium citrate, 0.5% (w/v) isoleucine, 0.7% (w/v) glycine, 1.6% (w/v) arginine hydrochloride, 1.2% (W/V) sodium glutamate, 0.03% (w/v) Polysorbate 80, 1.0% glycerol at pH 6.7 to 6.9. A fibrinogen solution II is one that is obtained by diluting the fibrinogen solution I by two-fold with water for injection to give a solution of about 6.0% (w/v) fibrinogen, to which glycerol is added to make a solution of 1.0% glycerol. A fibrinogen solution III is one that is obtained by diluting the fibrinogen solution I by four-fold with water for injection to give a solution of about 3.0% (w/v) fibrinogen, to which glycerol is added to make a solution of 1.0% glycerol. For a lyophilized powder of thrombin, one from Bolheal (registered trademark) (THE CHEMO-SERO-THERAPEUTIC RESEARCH INSTITUTE) was used. For calcium chloride powder, calcium chloride dihydrate (nacalai tesque) was used. For a solution of powder dispersion, ethanol (Wako Pure Chemical Industries, Ltd.; purity: 99.5%) was used.

Bioabsorbable sheets of a size 2.5×2.0 cm were placed on Petri dishes. For a sheet-shaped support of a thickness 0.8 mm, Neoveil 03G and Neoveil 05G were piled up. For a sheet-shaped support of a thickness 0.45mm, Neoveil 015G and Neoveil 03G or three Neoveil 015G were piled up. For a sheet-shaped support of a thickness 0.3mm, Neoveil 03G was used or two Neoveil 015G were piled up.

For Inventions A, B, J, K and L, bioabsorbable sheets were soaked with a fibrinogen solution I at 50 µL/cm² of fibrinogen per area of the surface that holds fibrinogen and were lyophilized to prepare fibrinogen-holding sheets. For Inventions C and D, bioabsorbable sheets were soaked with a fibrinogen solution I at 100 µL/cm² of fibrinogen per area of the surface that holds fibrinogen and were lyophilized to prepare fibrinogen-holding sheets. For Invention E, a bioabsorbable sheet was soaked with a fibrinogen solution III at 50 µL/cm² of fibrinogen per area of the surface that holds fibrinogen and was lyophilized to prepare a fibrinogen-holding sheet. For Invention F, a bioabsorbable sheet was soaked with a fibrinogen solution III at 100 µL/cm² of fibrinogen per area of the surface that holds fibrinogen and was lyophilized to prepare a fibrinogen-holding sheet. For Invention G, a bioabsorbable sheet was soaked with a fibrinogen solution II at 50 µL/cm² of fibrinogen per area of the surface that holds fibrinogen and was lyophilized to prepare a fibrinogen-holding sheet.

The surface of the respective fibrinogen-holding sheets opposite to the surface that holds fibrinogen was applied with a powder solution of a lyophilized powder of thrombin and calcium chloride powder dispersed in ethanol and the sheets were dried. An amount of a lyophilized powder of thrombin to be applied per area of the surface that holds thrombin was as follows: 13.8U/cm² for Inventions A, B, C, D, E, F and G; 0.28U/cm² for Invention J; 1.38U/cm² for Invention K; and 4.6U/cm² for Invention L. An amount of calcium chloride powder to be applied was 0.32 mg/cm².

The conditions of the respective Inventions are summarized in the table below.

**Table 1**

| *1 | *2 | *3 | *4 | *5 | *6 |
|---|---|---|---|---|---|
| A | 0.3 | I(12%) | 50 | 6 | 13.8 |
| B | 0.45 | I(12%) | 50 | 6 | 13.8 |
| C | 0.45 | I(12%) | 100 | 12 | 13.8 |
| D | 0.8 | I(12%) | 100 | 12 | 13.8 |
| E | 0.3 | III(3%) | 50 | 1.5 | 13.8 |
| F | 0.3 | III(3%) | 100 | 3 | 13.8 |
| G | 0.3 | II(6%) | 50 | 3 | 13.8 |
| J | 0.3 | I(12%) | 50 | 6 | 0.28 |
| K | 0.3 | I(12%) | 50 | 6 | 1.38 |
| L | 0.3 | I(12%) | 50 | 6 | 4.6 |

| | | | | | |
|---|---|---|---|---|---|
| *1: Invention *2: A thickness of sheet-shaped support (mm) *3: Fibrinogen solution (fibrinogen conc. w/v) *4: An amount of fibrinogen solution (µL/cm² ) *5: An amount of fibrinogen (mg/cm²) *6: An amount of thrombin (U/cm²) | | | | | |

### Example 2: Test for evaluating adhesive force of sheet preparation

For saline, saline of Japanese Pharmacopoeia (Otsuka Pharmaceutical Co., Ltd.) was used. For water for injection, water for injection of Japanese Pharmacopoeia (The Chemo-Sero-Therapeutic Research Institute) was used. As a control of a sheet preparation, TachoComb (registered trademark) (CSL Behring) was used.

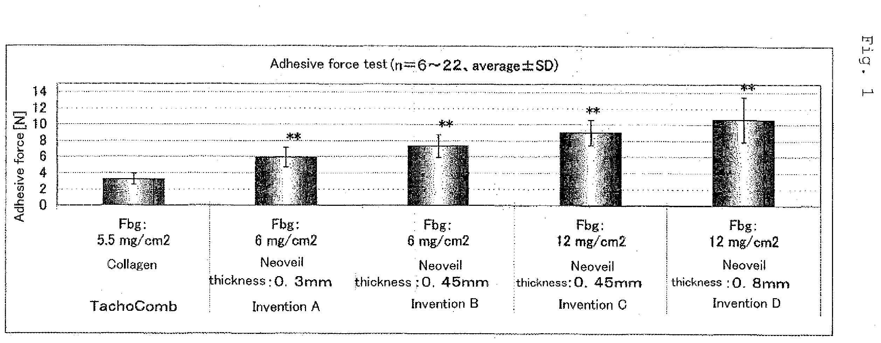

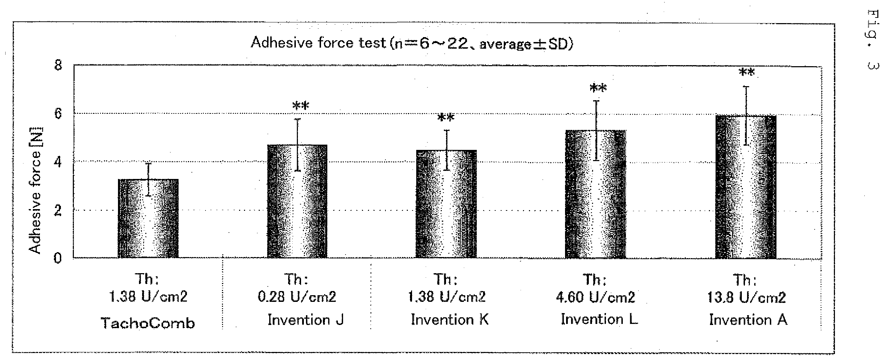

Two sheets of pig dermis were fixed by fixtures separately (adhesion area: 2.5×2.5 cm), 50 µL/cm² of saline was applied to the sheets, and a sheet preparation was placed thereon. After pressure for five minutes, one of the fixtures was immediately pulled horizontally, a maximum tensile strength (N: Newton) was determined and the obtained value was recorded as an adhesive force of the sheet preparation. N number was 22 for TachoComb, 5 for Inventions E/G, 6 for Inventions A/B/D/F/J/K/L, and 8 for Invention D. The results are shown in Figs. 1, 2 and 3.

As is clear from Figs. 1, 2 and 3, the adhesive force of Inventions showed significant difference from TachoComb by U-test of Mann-Whitney (**P<0.01). Therefore, it was proved that the sheet preparation according to the present invention exhibited more potent tissue-adhesive, sealing and hemostatic effects than those of the preparation currently clinically approved.

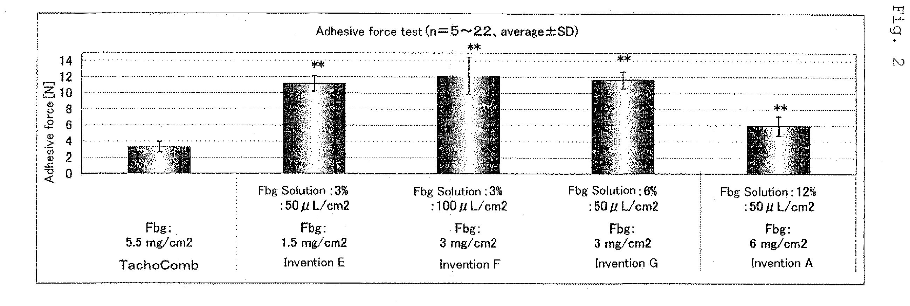

Besides, in Fig. 2, Inventions E, F and G exhibited more excellent adhesive effect than Invention A in spite of a smaller amount of fibrinogen applied to a sheet-shaped support for Inventions E, F and G. Although it can easily be conceived that the presence of a higher concentration of fibrinogen on a sheet-shaped support would provide a higher adhesive force, it was demonstrated that a factor other than an amount of fibrinogen on a sheet-shaped support (i.e. a concentration of a fibrinogen solution to be applied) would also contribute to an adhesive force, which was a surprising finding to the present inventors.

### Example 3: SDS-PAGE of sheet preparation

### [Preparation of samples before gelation: 0 minute]

Tested were TachoComb and Invention B prepared in Example 1. Sheets of 0.5×0.8cm=0.4cm² were prepared and placed in a sample tube. A solution for reduction was added to the sample tube. While properly stirring the solution containing the sheets, the sheets were dissolved at room temperature for 48 hours. After boiling, phoresis, staining (CBB) and discoloring were carried out.

### [Preparation of samples after gelation: 5, 60 minutes]

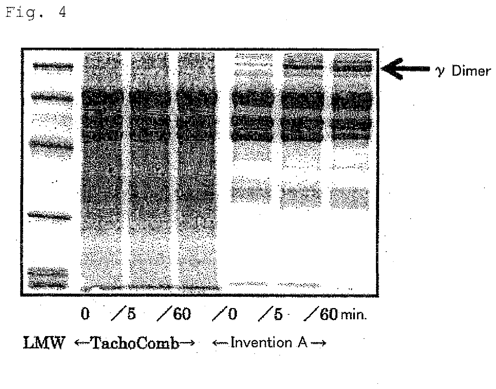

Tested were TachoComb and Invention A prepared in Example 1. Sheets of 0.5×0.8cm=0.4cm² were prepared and placed in a sample tube. Saline (40 µL) was added to the sample tube to dissolve and to be subject to gelation. Five minutes or sixty minutes after addition of saline and gelation, a solution for reduction was added to quench the gelation. While properly stirring the solution containing the gel sheets, the sheets were dissolved at room temperature for 48 hours. After boiling, phoresis, staining (CBB) and discoloring were carried out. The results are shown in Fig. 4.

As is clear from Fig. 4, Inventions formed a γ dimer which the conventional preparation could not form. Therefore, it was proved that the sheet preparation according to the present invention had enhanced physical strength and stability of fibrin clot due to formation of cross-linking within fibrin gel-matrix to thereby exhibit potent tissue-adhesive, sealing and hemostatic effects.

### Example 4: Test for evaluating hemostatic effect of sheet preparation

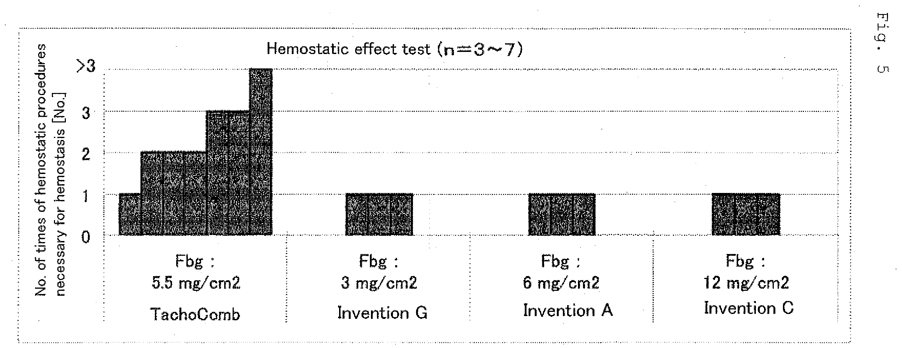

An animal model for evaluating hemostatic effect was prepared using SD male rat of 10-12 weeks old. Under anesthesia with xylazine hydrochloride and ketamine hydrochloride, the abdomen was incised along the median line. The abdominal aorta was exposed using gauze. Sodium heparin was administered via the abdominal vena cava at 300 U/kg. Two minutes after administration of heparin, the abdominal aorta was punctured with an injection needle to cause bleeding. Immediately thereafter, groups of test sheets of 1×1cm were applied and pressed for 3 minutes. After pressure, the occurrence of rebleeding was observed for 3 minutes. When rebleeding was observed, the test sheet group was removed and a new test sheet group was applied and the hemostatic procedure was carried out. The hemostatic effect was evaluated with a number of times of the hemostatic procedures necessary for hemostasis. N number was 7 for TachoComb, and 3 for Inventions A/C/G. The results are shown in Fig. 5.

As is clear from Fig. 5, inventions had a higher hemostatic effect than TachoComb. Therefore, it was proved that the sheet preparation according to the present invention exhibited more potent tissue-adhesive, sealing and hemostatic effects than those of the preparation currently clinically approved.

### INDUSTRIAL APPLICABILITY

The sheet preparation according to the present invention is a very convenient pharmaceutical preparation that does not require time for preparation and may be used in case of emergency for attaining tissue adhesion, sealing and hemostasis during surgical operation. It may exert more potent tissue-adhesive, sealing and hemostatic effects than the currently available pharmaceutical preparations and may form a γ-dimer, which the currently available pharmaceutical preparations cannot form, to thereby enhance physical strength and stability of fibrin clot. Besides, since it can more easily be prepared than the prior art, a sheet preparation that is inexpensive and highly effective may be provided. The present invention may provide a sheet preparation that is highly effective, convenient and inexpensive and a process for preparing the same.

## Claims

1. A sheet preparation **characterized by** that fibrinogen is held on one surface of a sheet-shaped support and thrombin is held on the other surface of the sheet-shaped support where fibrinogen is not held and that fibrinogen and thrombin are held separately from each other, wherein fibrinogen is a lyophilized powder of fibrinogen which is obtainable by applying or soaking a fibrinogen solution to one surface of the sheet-shaped support and then lyophilizing the sheet-shaped support, wherein said fibrinogen solution is one that contains 3% to 6% (w/v) fibrinogen, and wherein the sheet-shaped support is one made from polyglycolic acid and having a thickness of at least 0.3 mm.

2. The sheet preparation according to claim 1 wherein said sheet-shaped support is one made from a non-woven fabric prepared by needle-punching a knit or a textile of polyglycolic acid.

3. The sheet preparation according to claim 1 or 2 wherein said fibrinogen is held on the surface of the sheet-shaped support at 1.5 mg/cm² or more per area of the surface that holds fibrinogen.

4. The sheet preparation according to any of claims 1 to 3 wherein thrombin is a thrombin powder.

5. The sheet preparation according to claim 4 wherein said thrombin powder is one obtainable by applying a powder dispersion in which a thrombin powder is dispersed in a solvent to the other surface of the sheet-shaped support where fibrinogen is not held and drying the sheet-shaped support.

6. The sheet preparation according to claim 5 wherein said solvent is an alcohol.

7. The sheet preparation according to claim 6 wherein said alcohol is selected from the group consisting of ethanol, methanol, isopropyl alcohol, 1-butanol, 2-butanol, isobutyl alcohol, and isopentyl alcohol.

8. The sheet preparation according to claim 7 wherein said alcohol is ethanol.

9. The sheet preparation according to any of claims 1 to 8 wherein said thrombin is held on the surface of the sheet-shaped support at 0.28 U/cm² or more per area of the surface that holds thrombin.

10. The sheet preparation according to any of claims 1 to 9 wherein said sheet preparation comprises at least one member selected from the group consisting of Factor XIII, aprotinin, albumin, calcium chloride, a nonionic surfactant, and various amino acids.

11. A process for preparing the sheet preparation as set forth in any of claims 1 to 10, said process comprising holding fibrinogen on one surface of a sheet-shaped support and holding thrombin on the other surface of the sheet-shaped support where fibrinogen is not held.

12. A process for preparing a sheet preparation according to claim 11, which comprises the steps of:
(A) applying or soaking one surface of a sheet-shaped support with a fibrinogen solution and lyophilizing the sheet-shaped support to thereby let a lyophilized powder of fibrinogen be held on the sheet-shaped support; and
(B) applying a powder dispersion of a thrombin powder to the other surface of the sheet-shaped support and drying the sheet-shaped support to thereby let a thrombin powder be held on the sheet-shaped support;
wherein fibrinogen and thrombin are separately held on the distinct surfaces of the sheet-shaped support.

13. The process for preparing a sheet preparation according to claim 12 wherein said fibrinogen solution is applied in such an amount that said fibrinogen solution does not reach the other surface of the sheet-shaped support where a thrombin powder is held.

14. The sheet preparation as set forth in any of claims 1 to 10 for use in hemostasis.

## Patentansprüche

1. Ein Folienpräparat, **dadurch gekennzeichnet, dass** Fibrinogen auf einer Oberfläche eines folienförmigen Trägers gehalten wird und Thrombin auf der anderen Oberfläche des folienförmigen Trägers gehalten wird, wo Fibrinogen nicht gehalten wird, und dass Fibrinogen und Thrombin getrennt voneinander gehalten werden, wobei Fibrinogen ein lyophilisiertes Pulver aus Fibrinogen ist, welches durch Auftragen oder Tränken einer Fibrinogenlösung auf eine Oberfläche des folienförmigen Trägers und dann Lyophilisieren des folienförmigen Trägers erhältlich ist, wobei die Fibrinogenlösung eine ist, welche 3% bis 6% (w/v) Fibrinogen enthält, und wobei der folienförmige Träger einer ist, der aus Polyglykolsäure hergestellt ist und eine Dicke von mindestens 0,3 mm aufweist.

2. Das Folienpräparat gemäß Anspruch 1, wobei der folienförmige Träger einer ist, der aus einem Vliesstoff, hergestellt durch Vernadelung einer Maschenware oder eines Textils aus Polyglykolsäure, hergestellt ist.

3. Das Folienpräparat gemäß Anspruch 1 oder 2, wobei das Fibrinogen auf der Oberfläche des folienfönnigen Trägers bei 1,5 mg/cm² oder mehr pro Fläche der Oberfläche, die Fibrinogen hält, gehalten wird.

4. Das Folienpräparat gemäß einem der Ansprüche 1 bis 3, wobei Thrombin ein Thrombinpulver ist.

5. Das Folienpräparat gemäß Anspruch 4, wobei das Thrombinpulver eines ist, das durch Auftragen einer Pulverdispersion, in welcher ein Thrombinpulver in einem Lösungsmittel dispergiert ist, auf die andere Oberfläche des folienförmigen Trägers, wo Fibrinogen nicht gehalten wird, und Trocknen des folienförmigen Trägers erhältlich ist.

6. Das Folienpräparat gemäß Anspruch 5, wobei das Lösungsmittel ein Alkohol ist.

7. Das Folienpräparat gemäß Anspruch 6, wobei der Alkohol ausgewählt ist aus der Gruppe bestehend aus Ethanol, Methanol, Isopropylalkohol, 1-Butanol, 2-Butanol, Isobutylalkohol und Isopentylalkohol.

8. Das Folienpräparat gemäß Anspruch 7, wobei der Alkohol Ethanol ist.

9. Das Folienpräparat gemäß einem der Ansprüche 1 bis 8, wobei das Thrombin auf der Oberfläche des folienförmigen Trägers bei 0,28 U/cm² oder mehr pro Fläche der Oberfläche, die Thrombin hält, gehalten wird.

10. Das Folienpräparat gemäß einem der Ansprüche 1 bis 9, wobei das Folienpräparat mindestens ein Element ausgewählt aus der Gruppe bestehend aus Faktor XIII, Aprotinin, Albumin, Calciumchlorid, einem nichtionischen Tensid und verschiedenen Aminosäuren umfasst.

11. Ein Verfahren zur Herstellung des Folienpräparats wie in einem der Ansprüche 1 bis 10 definiert, wobei das Verfahren das Halten von Fibrinogen auf einer Oberfläche eines folienförmigen Trägers und das Halten von Thrombin auf der anderen Oberfläche des folienförmigen Trägers, wo Fibrinogen nicht gehalten wird, umfasst.

12. Ein Verfahren zur Herstellung eines Folienpräparats gemäß Anspruch 11, welches die Schritte umfasst:
(A) Auftragen oder Tränken einer Oberfläche eines folienförmigen Trägers mit einer Fibrinogenlösung und Lyophilisieren des folienförmigen Trägers, um dadurch ein lyophilisiertes Pulver aus Fibrinogen auf dem folienförmigen Träger zu halten; und
(B) Auftragen einer Pulverdispersion eines Thrombinpulvers auf die andere Oberfläche des folienförmigen Trägers und Trocknen des folienförmigen Trägers, um dadurch ein Thrombinpulver auf dem folienförmigen Träger zu halten;
wobei Fibrinogen und Thrombin getrennt voneinander auf den unterschiedlichen Oberflächen des folienförmigen Trägers gehalten werden.

13. Das Verfahren zur Herstellung eines Folienpräparats gemäß Anspruch 12, wobei die Fibrinogenlösung in einer solchen Menge aufgetragen wird, dass die Fibrinogenlösung die andere Oberfläche des folienförmigen Trägers, wo ein Thrombinpulver gehalten wird, nicht erreicht.

14. Das Folienpräparat wie in einem der Ansprüche 1 bis 10 definiert, zur Verwendung bei der Hämostase.

## Revendications

1. Préparation de feuille **caractérisée en ce que** du fibrinogène est maintenu sur une surface d'un support en forme de feuille et de la thrombine est maintenue sur l'autre surface du support en forme de feuille où du fibrinogène n'est pas maintenu et **en ce que** le fibrinogène et la thrombine sont maintenus séparément l'un de l'autre, où le fibrinogène est une poudre de fibrinogène lyophilisée qui peut être obtenue par application à ou trempage d'une surface du support en forme de feuille avec une solution de fibrinogène puis lyophilisation du support en forme de feuille, où ladite solution de fibrinogène est une solution qui contient 3 % à 6 % (p/v) de fibrinogène, et où le support en forme de feuille est un support produit à partir de poly(acide glycolique) et ayant une épaisseur d'au moins 0,3 mm.

2. Préparation de feuille selon la revendication 1 où ledit support en forme de feuille est un support produit à partir d'une étoffe non tissée préparée par aiguilletage d'un tricot ou d'un textile de poly(acide glycolique).

3. Préparation de feuille selon la revendication 1 ou 2 où ledit fibrinogène est maintenu sur la surface du support en forme de feuille à raison de 1,5 mg/cm² ou plus par aire de la surface qui maintient du fibrinogène.

4. Préparation de feuille selon l'une quelconque des revendications 1 à 3 où la thrombine est une poudre de thrombine.

5. Préparation de feuille selon la revendication 4 où ladite poudre de thrombine est une poudre qui peut être obtenue par application d'une dispersion de poudre dans laquelle une poudre de thrombine est dispersée dans un solvant à l'autre surface du support en forme de feuille où du fibrinogène n'est pas maintenu et séchage du support en forme de feuille.

6. Préparation de feuille selon la revendication 5 où ledit solvant est un alcool.

7. Préparation de feuille selon la revendication 6 où ledit alcool est choisi dans le groupe consistant en l'éthanol, le méthanol, l'alcool isopropylique, le 1-butanol, le 2-butanol, l'alcool isobutylique et l'alcool isopentylique.

8. Préparation de feuille selon la revendication 7 où ledit alcool est l'éthanol.

9. Préparation de feuille selon l'une quelconque des revendications 1 à 8 où ladite thrombine est maintenue sur la surface du support en forme de feuille à raison de 0,28 U/cm² ou plus par aire de la surface qui maintient de la thrombine.

10. Préparation de feuille selon l'une quelconque des revendications 1 à 9 où ladite préparation de feuille comprend au moins un membre choisi dans le groupe consistant en le facteur XIII, l'aprotinine, l'albumine, le chlorure de calcium, un tensioactif non ionique et différents acides aminés.

11. Procédé pour préparer la préparation de feuille selon l'une quelconque des revendications 1 à 10, ledit procédé comprenant le maintien de fibrinogène sur une surface d'un support en forme de feuille et le maintien de thrombine sur l'autre surface du support en forme de feuille où du fibrinogène n'est pas maintenu.

12. Procédé pour préparer une préparation de feuille selon la revendication 11, qui comprend les étapes de :
(A) application à ou trempage d'une surface d'un support en forme de feuille avec une solution de fibrinogène et lyophilisation du support en forme de feuille pour amener ainsi une poudre de fibrinogène lyophilisée à être maintenue sur le support en forme de feuille ; et
(B) application d'une dispersion de poudre d'une poudre de thrombine à l'autre surface du support en forme de feuille et séchage du support en forme de feuille pour amener ainsi une poudre de thrombine à être maintenue sur le support en forme de feuille ;
où le fibrinogène et la thrombine sont maintenus séparément sur les surfaces distinctes du support en forme de feuille.

13. Procédé pour préparer une préparation de feuille selon la revendication 12 où ladite solution de fibrinogène est appliquée en une quantité telle que ladite solution de fibrinogène n'atteint pas l'autre surface du support en forme de feuille où une poudre de thrombine est maintenue.

14. Préparation de feuille selon l'une quelconque des revendications 1 à 10 destinée à être utilisée dans l'hémostase.
